(19) **Europäisches Patentamt / European Patent Office / Office européen des brevets**

(11) **EP 4 115 946 B1**

(12) **EUROPEAN PATENT SPECIFICATION**

(45) Date of publication and mention of the grant of the patent:
**18.06.2025  Bulletin 2025/25**

(21) Application number: **21184266.1**

(22) Date of filing: **07.07.2021**

(51) International Patent Classification (IPC):
***A61N 1/372*** (2006.01)

(52) Cooperative Patent Classification (CPC):
**A61N 1/37252**

(54) **COMMUNICATION SYSTEM AND COMMUNICATION METHOD FOR WIRELESS DATA TRANSMISSION BETWEEN AN IMPLANT AND AN EXTERNAL DEVICE**

KOMMUNIKATIONSSYSTEM UND KOMMUNIKATIONSVERFAHREN ZUR DRAHTLOSEN DATENÜBERTRAGUNG ZWISCHEN EINEM IMPLANTAT UND EINER EXTERNEN VORRICHTUNG

SYSTÈME ET PROCÉDÉ DE COMMUNICATION POUR LA TRANSMISSION DE DONNÉES SANS FIL ENTRE UN IMPLANT ET UN DISPOSITIF EXTERNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**11.01.2023  Bulletin 2023/02**

(73) Proprietor: **BIOTRONIK SE & Co. KG**
**12359 Berlin (DE)**

(72) Inventors:
• **ELSNER, Joachim**
 **14059 Berlin (DE)**
• **KÖPPEL, Jürgen**
 **85599 Parsdorf (DE)**
• **BIERSCHNEIDER, Emanuel**
 **12459 Berlin (DE)**
• **GARTZ, Wolfgang**
 **15528 Spreenhagen (DE)**
• **KAMENZ, Uwe**
 **13507 Berlin (DE)**
• **STÜRMER, Uwe**
 **10119 Berlin (DE)**
• **STEPHAN, Enrico**
 **13055 Berlin (DE)**
• **GORECKI, Gunnar**
 **12101 Berlin (DE)**

(74) Representative: **Biotronik Corporate Services SE**
**Corporate Intellectual Property**
**Sieversufer 7-9**
**12359 Berlin (DE)**

(56) References cited:
**WO-A1-2018/160451    US-A1- 2002 045 920**
**US-A1- 2003 009 204    US-A1- 2019 013 087**

**Description**

The invention is directed to a communication system for wireless data transmission between an implantable medical device (IMD, implant) and an external device wherein the IMD is configured to monitor the health status of a patient and/or configured to deliver a therapy signal to the patient. The external device is located at least partially extracorporeally. The invention is further directed to a respective communication method for wireless data transmission, a respective computer program product and a respective computer readable data carrier.

Active and passive implantable medical devices (IMDs, implants), for example, a pacemaker (with leads), a BioMonitor, an Implantable Leadless Pacer (ILP), an Implantable Leadless Pressure Sensor (ILPS), an Implantable Cardiac Defibrillator (ICD) or a Shockbox, contain sensors that collect physiological signals in order to monitor the health status of the patient and transmit them as data to an external device (e.g. smartphone, computer, remote server) using the communication unit. The data collected from these various sensors can include, but are not limited to, ECG, impedance, activity, posture, heart sounds, pressure, respiration data and other data. Active IMDs, e.g. pacemaker, ILP, ICD, or Shockbox may provide therapy signals to the patient, for example electrical stimulation within a heart chamber or atrium.

Usually, such IMD comprises a processor for data processing and a transceiver module configured to bi-directionally exchange messages with a communication unit of the external device if, for example, implanted within the body of the patient. The communication unit of the external device which is located at least partly extracorporeally is configured for bi-directional data transmission using the transceiver module of the IMD. The communication unit produces and sends messages to the transceiver module of the IMD in form of requests, for example for receiving data concerning the health status of the patient or IMD status from the IMD or for programming (in order to configure the IMD to apply appropriate therapies to the patient).

Document US 2003/0009204 A1 describes a system and method for optimizing short and long-range telemetry communications between an implantable medical device and an external device such as an external programmer or remote monitor. In accordance with the invention, data is transmitted from the implantable device to the external device at either a higher or lower data rate.

Document US 2018/0152972 A1 describes a method and system for data transmission, wherein a communication link is initiated between an external instrument and an IMD. In a first connection interval data packets are conveyed between the external instrument and the IMD a connection criteria is monitored that includes at least one of a data throughput requirement. Further, it is changed from a first connection interval to a second connection interval based on the connection criteria in order to increase the longevity of the IMD, wherein the second connection interval may be longer than the first connection interval, wherein the changing operation comprises changing to the second connection interval when the data throughput requirement drops below a data threshold.

The above document shows that the parameters of a communication link need to be considered in order to avoid undue drain of IMD battery. This is particularly true for telemetry communication between the IMD if implanted in a patient's body and the external device. Often, such telemetry communication is realized as short communication connections comprising exchange of a few data packets which is repeated in pre-defined, mostly regular time intervals.

Usually, telemetry communication between IMD and the external device uses a low link margin. In such case a high data rate causes communication loss and potentially high repeat rate leading to increased energy consumption. Since the short-time communication connections contain only a few data packets, usual "negotiation" of the optimum data rate between the communication partners would increase energy consumption, as well. Usual quality of service (QoS) methods for determining the overall performance of a data transmission service comprising requirements on all the aspects of a connection, such as service response time, loss, signal-to-noise ratio, crosstalk, echo, interrupts, frequency response, loudness levels, etc. are ineffective methods for a short communication such as a IMD and external device communication in case of telemetry communication.

Accordingly, it is desirable to provide a communication system and method that does not use the IMD battery energy up and provides a fast result with regard to at least one optimal communication parameter leading to increase of longevity.

The above problem is solved by a communication system for a wireless data transmission between an IMD and an external communication unit with the features of claim 1, by a respective communication method with the features of claim 8, by a computer program product with the features of claim 13, and by a computer readable data carrier with the features of claim 14.

The problem is solved, in particular, by a communication system for wireless data transmission between an IMD and an external device comprising a communication unit and a processor, wherein the IMD is configured to monitor a bodily parameter of a patient and/or is configured to deliver a therapy signal to the patient, wherein the IMD comprises a transceiver module configured to exchange the data with the communication unit of the external device in an uplink direction from the transceiver module to the communication unit and in a downlink direction from the communication unit to the transceiver module, wherein the processor of the external device is configured to determine the signal strength of at least one data packet received by the communication unit and sent by the transceiver module of the implanted IMD with a pre-determined initial data rate and to determine the noise level of the communication unit, wherein the processor is further configured to determine a highest possible uplink data rate value for data transmission in the uplink direction and a highest possible downlink data rate value for data transmission in the downlink direction based on the immediately previously determined signal strength of the at least one data packet received by the communication unit from the transceiver module and the current noise level of the communication unit.

The IMD is an implantable medical device as defined above configured to monitor the health status of a patient and/or configured to deliver a therapy signal to the patient, for example an ILP, an ILPS or an ICD. Other examples for the IMD which may realize the above subject matter are the above mentioned IMD, an SCS device, a drug delivery device or a cochlear implant. The IMD is at least partly implantable within the patient's body. After implantation the IMD provides telemetry communication with the external device.

The IMD comprises a processor for data processing and a transceiver module (e.g. an antenna) for sending and transmitting messages (i.e. communication signals) to the communication unit as well as receiving messages from the communication unit. The processor of the IMD is electrically connected to the transceiver module and the processor of the external device is electrically connected to the communication unit. Alternatively, the transceiver module or the communication unit may be integrated within the processor of the IMD or the processor of the external device, respectively. The transceiver module and the communication unit are configured to send signals/messages in form of data packets to the respective other module/unit. Generally, such data packets are bitstrings embedded in a system of syntax rules and semantics rules defined in a communication protocol which are expressed in respective algorithms and data structures. The messages received by the transceiver module are transmitted to the respective processor for data processing. Analogously, the processor produces the content of the signals/messages which are transmitted by the transceiver module or communication unit to the respective partner.

The memory module of the IMD may include any volatile, non-volatile, magnetic, or electrical media, such as a random access memory (RAM), read-only memory (ROM), non-volatile RAM (NVRAM), electrically-erasable programmable ROM (EEPROM), flash memory, or any other memory device.

The IMD may comprise further modules such as a power supply such as a battery, at least one sensor for retrieving physiological signals from the patient and/or a signal generator for generating, for example, electrical or electromagnetically therapy signals in order to provide the therapy to the patient. The transceiver module, the memory module, the power supply, the at least one sensor and/or the signal generator may be electrically connected to the processor. The components of the IMD may be located within a hermetically sealed housing.

The external device is always located at least partially extracorporeally, in particular in the situation in which the IMD is implanted in the patient's body. The external device may be a computer, a smartphone, a server or similar computing device comprising a communication unit and a processor. The external device may be a so-called patient remote device (PR) which may serve as a transceiver to route communication between the IMD and a remote monitoring server. The PR may give the patient or an HCP the ability to change the active therapy program of the IMD, control its stimulation amplitude, turn stimulation on/off, and view battery status. Accordingly, it may also serve as an external programmer for the IMD. The described functionality of the external device and below method may be realized by a corresponding app.

The communication unit is configured for bi-directional communication with the transceiver module of the IMD and therefore comprises a transceiver for messages (signals), for example, an antenna.

For data/signal processing the external device and the IMD each comprise or may comprise a dedicated processor which is generally regarded as a functional unit of external device or the IMD, that interprets and executes instructions comprising an instruction control unit and an arithmetic and logic unit. The processor may comprise a microprocessor, a controller, a digital signal processor (DSP), an application specific integrated circuit (ASIC), a field-programmable gate array (FPGA), discrete logic circuitry or any combination thereof. Alternatively or additionally, the processor may be realized using integrated dedicated hardware logic circuits, in particular in the case of an IMD due to the small size and extreme power limitation. The processor may comprise a plurality of processing subunits.

Wireless communication of the communication unit and the transceiver module of the IMD comprises communication over the air (without wire) using electromagnetic waves, for example, MedRadio/MICS/MEDS, EDGE, EV-DO, Flash-OFDM, GPRS, HSPA, LoRaWAN, RTT, UMTS, Narrowband IoT, Bluetooth, WLAN (WiFi), ZigBee, NFC, LTE, Wireless USB, Wibree (BLE), Ethernet or WiMAX in the radio frequency region, or IrDA or free-space optical communication (FSO) in the infrared or optical frequency region. Accordingly, the respective communication protocols, often with the same name (i.e. a system of rules that allows two or more entities of a communications system to transmit information via a kind of variation of a physical quantity, defining rules, syntax, semantics and synchronization of communication and possible error recovery methods and being implemented by hardware, software or a combination of both) or cooperating protocols (protocol suites) may be used, for example TCP/IP protocol suite (e.g. IPv6, TCP, UDP, SMTP, HTTP/2) also with TLS or SSL, IPX/SPX, X.25, AX.25, ebXML, Apple Talk, Bluetooth protocols (e.g. BR/EDR), Bluetooth 4.0 (BLE), ZigBee protocol, NFC protocol, IEEE 802.11 and 802.16 protocols, etc.. After implantation of the IMD the communication path comprises a path section within the tissue of the patient's body.

As indicated above, the communication of the communication unit and the transceiver module may use the MedRadio/MICS/MEDS specification. The Medical Device Radiocommunications Service (MedRadio) is a specification and communication spectrum created for the U.S. Federal Communications Commission (FCC) for the communication needs of diagnostic and therapeutic IMD and body-worn medical devices. The covered spectrum comprises frequency bands in the range 401 MHz to 406 MHz and 413 MHz to 457 MHz as well as in the range 2360 MHz to 2400 MHz. The specification and nearly identical spectrum have also been created by the European Telecommunications Standards Institute (ETSI), with the specification largely referred to as MICS/MEDS (Medical Data Service) in Europe and other parts of the world. The MICS band may be divided into 25 kHz channels, wherein an IMD transceiver may take up to 300 kHz. Data rates of up to 800 kbit/s are possible in this case. The transmitted power is very low at 25 μW EIRP (-16 dBm) to avoid interference.

Accordingly, the communication module and the transceiver module comprise the respective hardware and software adapted to the used communication techniques / communication protocols.

With regard to the invention each processor is regarded as a functional unit of the IMD and the external device, respectively, that interprets and executes instructions comprising an instruction control unit and an arithmetic and logic unit. The remote computer is a functional unit that can perform substantial computations, including numerous arithmetic operations and logic operations without human intervention, such as, for example, a personal mobile device (PMD), a desktop computer, a server computer, clusters/warehouse scale computer or embedded system.

The above communication system has the advantage that the highest possible uplink data rate value and the highest possible downlink data rate value for a pre-defined bit-error rate (BER), for example BER = $10^{-5}$ or BER = $10^{-3}$, are determined based on determined values of the external device only. There is no (additional) determination of any value within the IMD needed. Additionally, the determination of the highest possible uplink data rate value and the highest possible downlink data rate value is possible after receipt of only one data packet by the communication unit from the IMD which makes the determination very fast and leads to reduction of energy consumption of the IMD and therefore increases longevity.

In one embodiment, the highest possible uplink data rate values for data transmission in the uplink direction and in the downlink direction consider a BER of $10^{-3}$.

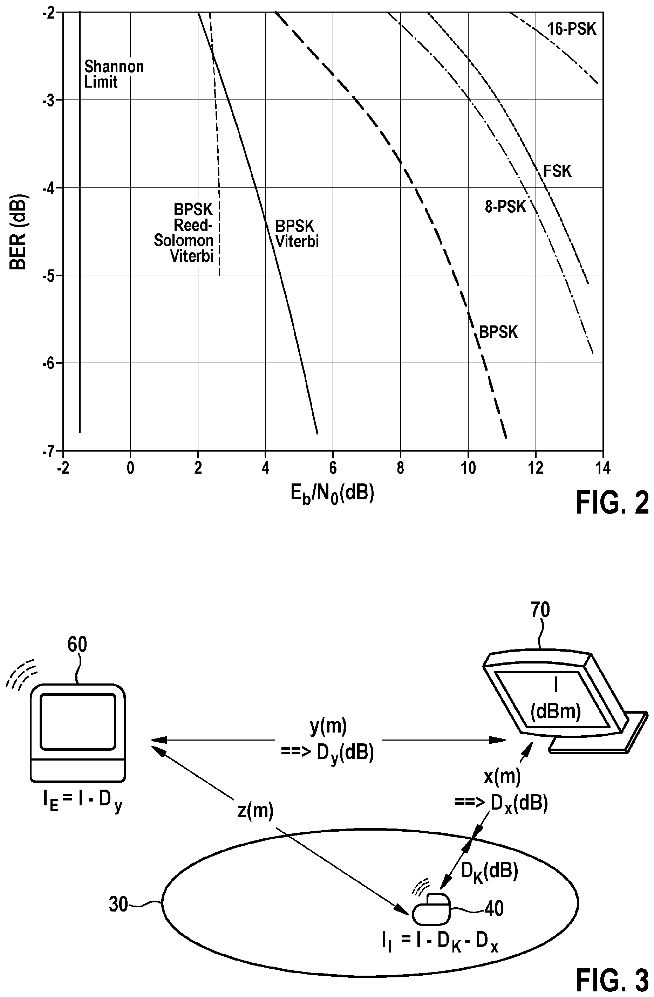

The inventors have recognized that the highest possible data rate for a given BER and communication connection depends on a received carrier to noise ratio measure. Accordingly, the inventors determine the signal and inference level received by the communication unit of the external device in order to receive a value for the highest possible data rate. Fig. 2 shows the BER drawn over the normalized carrier-to-noise ratio measure denoted Eb/N0 (energy per bit to noise power spectral density ratio) for different types of bit error rate tests (BERT). One can derive from this diagram, for example, that for FSK modulation and BER = $10^{-5}$ the band width and thereby the data rate can be increased if Eb/N0 > 14 dB.

The determination of the highest possible uplink data rate and the highest possible downlink data rate may further be based on the used transceiver module of the IMD. Each transceiver chip type realizes a certain sensitivity for each data rate in relation to thermal noise N. As an example the following table (table 1) lists the sensitivity with regard to the data rate for a typical transceiver chip.

| data rate | sensitivity at thermal noise N |
|-----------|-------------------------------|
| 200 kb/s  | -104 dBm                      |
| 65 kb/s   | -106 dBm                      |
| 32 kb/s   | -109 dBm                      |
| 16 kb/s   | -112 dBm                      |
| 4 kb/s    | -116 dBm                      |

Accordingly, in one embodiment, the highest possible uplink data rate values for data transmission in the uplink direction and in the downlink direction consider a predetermined type of IMD transceiver module.

The inventors assume that the signal strength $C_E$ of one data packet received by the communication unit and sent by the transceiver module using a pre-defined initial data rate is

$$C_E = TX_I + L \quad \text{(formula 0),}$$

wherein $TX_I$ (in dBm) is the transmitter power output of the transceiver module of the IMD and L all losses of the transmission path. Further assuming the symmetry of the transmission path, the signal level $C_I$ of a data packet received by the transceiver module and sent by the communication unit is

$$C_I = TX_E + L = C_E + TX_E - TX_I \quad \text{(formula I),}$$

wherein $TX_E$ (in dBm) is the transmitter power output of the communication unit of the external device and L the (all) losses of the transmission path. The above formula is further based on the assumption that the same frequency is used for uplink and downlink data transmission, which is, for example, the case if the MICS band is used for data transmission. Accordingly, in one embodiment, the highest possible uplink data rate values for data transmission in the uplink direction and in the downlink direction consider a predetermined frequency band for data transmission.

After measurement of the signal strength $C_E$ of the signal of at least one received data packet and determination of $TX_I$ from the content of the respective data packet, the processor of the external device determines the signal level $C_I$ using Formula I. $TX_E$ is known from the external device for the pre-defined initial data rate.

As indicated above, the second important value with regard to the determination of the highest possible uplink data rate value for data transmission in the uplink direction and a highest possible downlink data rate value for data transmission in the downlink direction is the current noise level received in the external device. This current noise level $I_E$ (in dBm) is determined by the processor of the external device over a pre-defined time period for the used transmission channel with a pre-defined band width B (in Hz), for example over several seconds, as a moving arithmetic mean value. A moving geometric or harmonic mean value may be used, as well. The noise level $I_E$ may be determined continuously or in pre-defined time intervals, every 5 to 20 minutes, for a pre-defined time interval, for example 1 to 60 seconds. The current noise level $I_E$ is the most recent determined noise level value according to above method.

As indicated in Fig. 3 an interfering source I (in dBm) 70 produces a noise level $I_I$ in the transceiver module of the IMD 40 in a distance x (in m). $I_I$ is calculated as $I_I = I - D_x - D_K$, wherein $D_x$ (in dB) is the free path loss and $D_K$ is the attenuation of the patient's tissue, wherein the tissue of the patient 30 is a part of the communication path. $I_E$ is the noise level (in dBm) produced by the interfering source I in the external device 60 in a distance y (in m), wherein an isotropic antenna is assumed having an antenna gain of 0 dBm. Based on the finding, that $D_K$ is great compared with $D_x$ and based on the assumptions that an interference source is usually located not closer than 25 cm from the IMD (x > 0.25 m) and that the external device is usually located 2 m or closer to the IMD (z <= 2 m) the following calculation is provided:

$$I_I = I - D_x - D_K \text{ and } I_E = I - D_y$$

$$D_{y/x} = D_y - D_x = 20 * \log_{10}(y/x)$$

$$y/x < (z+x)/x$$

Hence,

$$D_{y/x} < 20 * \log10((z+x)/x) = 20 * \log_{10}((2\text{ m} + 0.25\text{ m})/0.25\text{ m}) = 19$$

so that $D_{y/x} < 19$. Accordingly, 19 is an upper limit of $D_{y/x}$.

Accordingly, the noise level $I_I$ may be calculated as follows

$$I_I = I_E - D_K + 19 \quad \text{(formula II)}$$

Formula II gives a useful and sufficiently precise ("sharp") value for interference $I_I$ expected at the IMD.

The value does not depend on the position of the interfering source I and is determined without the actual need of measuring interference in the IMD: measurements take only place in the external device which saves energy in the IMD.

This value for interference can be used to adjust the data rate $d_{DL}$ in the downlink direction. Note that for telemetry using an IMD we have $D_K >> D_{y/x} (=19)$ and therefore $I_I << I_E$ which means that interference is much lower in the IMD than in the external device. This suggests that if the data rate is determined mostly by interference one will chose $d_{DL} > d_{UL}$.

The highest possible uplink data rate value $d_{UL}$ for data transmission in the uplink direction is determined by the processor of the external device based on the determined values $C_E$, $I_E$ and N and represents the data rate in uplink direction at which the communication unit of the external device is able to decode the received signal with a pre-defined BER (for example $10^{-3}$ or $10^{-5}$). Analogously, the highest possible downlink data rate value $d_{DL}$ for data transmission in the downlink direction is determined by the processor of the external device based on the calculated values $C_I$, $I_I$ and N and represents the data rate in downlink direction at which the transceiver module of the IMD is able to decode the received signal with a pre-defined BER (for example $10^{-3}$ or $10^{-5}$). N is the thermal noise floor in the communication unit or the transceiver module which may be derived from the Boltzmann constant $k_B$ (in J/K), the band width B (in Hz) and the temperature T (in ° K) from N = 10 * $\log_{10}$ (kB * T * B) + 30.

The parameters N, $TX_E$, $D_K$ and B are known for each specific communication channel and the used specific band.

For example, for the MICS band (402 Hz to 405 Hz) one can assume the following parameters:

$T_{XE}$ (in dBm) = -16; $D_K$ (in dB) = 31.5; B (in Hz) = 00,000; N (in dBm) = - 119.

These values are close to the parameters determined by the study ITU-SA-1346 (see the following table 2).

| | Uplink (Implant ⇨ Programmer | Downlink (Programmer ⇨ Implant |
|---|---|---|
| Frequency | 403.5 MHz +/- 1.5 MHz | |
| Modulation type | FSK | |
| Receiver noise bandwith | 200 kHz | 25 kHz |
| Ambient noise at receiver input | 20 dB above kTB | ≡ kTB (due to tissue loss) |
| Receiver noise figure | 4 dB | 9 dB |
| Receiver noise floor | - 101 dBm | - 121 dBm |
| Receive antenna gain | 2 dBi | - 31.5 dBi |
| Required SNR (BER = 1E-5) | 14 dB | |
| Free space loss at 2 metres | 30.5 dB | |
| Fade margin[1] (with diversity) | 10 dB | |
| Excess loss[2] (polarization, etc.) | 15 dB | |
| Transmitt antenna gain | - 31.5 dBi | 2 dBi |
| Power into antenna | - 2 dBm | - 22 dBm |
| ERP | - 33.5 dBm (at body surface) | - 20 dBm[3] |

As the sensitivities for the transceiver chip listed in table 1 are provided at thermal noise N, these values need to be adapted by I/N (in dB) with regard to the noise level of the interfering source I. Additionally, a tolerance value needs to be considered with regard to $TX_I$, $TX_E$, $C_E$, $I_E$ which is determined to about 6 dB for the usage with regard to the IMD and external device signal transmission path. Accordingly, the sensitivities listed in table 1 must be reduced by a following value $\Delta$

$$\Delta_I = \max (I_I/N, 0) + 6 = \max (I_I+125, 6) \quad \text{(formula IIIa)}.$$

$$\Delta_E = \max (I_E/N, 0) + 6 = \max (I_E+125, 6) \quad \text{(formula IIIb)}.$$

From formulas IIIa,b follows:

$$C_{Ieff} \text{ (in dBm)} = C_I - \Delta_I \quad \text{(formula IVa)}$$

$$C_{Eeff} \text{ (in dBm)} = C_E - \Delta_E \quad \text{(formula IVb)}$$

The highest possible uplink data rate value $d_{UL}$ for data transmission in the uplink direction may then be derived by the processor from table 1 (stored in the processor or a connected data memory) based on $C_{Eeff}$ and the highest possible downlink data rate value $d_{DL}$ for data transmission in the downlink direction from table 1 based on $C_{Ieff}$. Accordingly, the highest possible uplink data rate value $d_{UL}$ and the highest possible downlink data rate value $d_{DL}$ are determined by the processor of the external device using the immediately previously determined signal strength $C_E$ of one data packet or several data packets received by the communication unit within a pre-defined time period ending immediately before and the determined current noise level $I_E$ of the communication unit. All other parameters and assumptions are generally known or are automatically provided to the communication unit ($TX_I$ from the content of the first data packet). Hence, measurements by the transceiver module of the IMD are not necessary.

In one embodiment, the communication unit of the external device is further configured to transmit the determined highest possible uplink data rate value $d_{UL}$ and the highest possible downlink data rate value $d_{DL}$ to the transceiver module of the IMD and the IMD is configured to set the uplink and downlink data rates subsequently usable by the transceiver module to the data rate values received from the communication unit. The communication unit may send these data rate values $d_{UL}$ and $d_{DL}$ with a second data packet to the transceiver module of the IMD subsequent to the receipt of the first data packet.

In one embodiment, the external device is configured to set the uplink and downlink data rates subsequently usable by the communication unit to the determined highest possible uplink and downlink data rate values, for example after sending these values to the transceiver module, e.g. with the second data packet.

In one embodiment, the processor of the external device is configured to determine a signal loss L in the data transmission in the uplink and downlink direction, which may be determined by the processor of the external device based on above formula 0. The transmitter power output of the transceiver module of the IMD $TX_I$ is received by the transmitted data signal of the IMD which is received by the external device. The information about $TX_I$ is contained within the data signal. Accordingly, $L = C_E - TX_I$, wherein the signal level $C_E$ is determined by the communication unit as described above.

In another embodiment, the noise level $I_E$ (in dBm) of the communication unit may be observed over a pre-determined longer time period (e.g. several days or weeks) in order to identify at least one subsection of this time period in which regularly the noise level $I_E$ (in dBm) is higher than in other subsections. For that the noise level is monitored over the whole pre-determined time period continuously or in pre-determined time intervals. Then, pattern are identified and a respective subsection of the pre-determined time period determined. If there is at least one subsection at which regularly a higher noise level $I_E$ is detected, the time specification of such subsection is transmitted to the IMD by the communication unit in order to avoid this at least one subsection for telemetry communication. This means that telemetry communication is preferably provided outside of the determined subsections with higher noise level $I_E$.

The above problem is further solved, for example, by a communication method (which is a computer-implemented method) for a wireless data transmission between an IMD and an external device comprising a communication unit and a processor, wherein the IMD monitors a bodily parameter of a patient and/or delivers a therapy signal to the patient after implantation within the patient's body, wherein the IMD comprises a transceiver module exchanging the data with the communication unit of the external device in an uplink direction from the transceiver module to the communication unit and in a downlink direction from the communication unit to the transceiver module, with the following steps:

- the processor of the external device determines the signal strength of at least one data packet received by the communication unit, which was sent by the transceiver module of the implanted IMD with a pre-determined initial data rate,
- the processor of the external device determines the noise level of the communication unit, and
- the processor determines a highest possible uplink data rate value $d_{UL}$ for data transmission in the uplink direction and a highest possible downlink data rate value $d_{DL}$ for data transmission in the downlink direction based on the immediately previously determined signal strength of the at least one data packet received by the communication unit from the transceiver module and the current noise level of the communication unit.

In more detail the method comprises the following steps, wherein many features of the method are already explained with regard to the communication system above. It is therefore referred to the above explanation also with regard to the communication method.

At first, at least one first data packet is sent by the transceiver module of the IMD to the communication unit of the external device with a pre-determined initial data rate, wherein the information about the transmission power output of the transceiver module is provided within the content of the at least one first data packet. Further, the communication unit of the external device determines the signal strength $C_E$ (in dBm) of the received at least one first data packet. If more than one single data packet is used, the signal strength $C_E$ (in dBm) is determined as a mean value (i.e. arithmetic mean) or a median of the at least two data packets by the communication unit or the processor of the external device. However, in most cases only a single received data packed from the IMD would be sufficient. Then, using formula I, the signal strength $C_I$ (in dBm) is determined by the processor of the external device, wherein $TX_E$ is known from the parameters of the communication unit of the external device and $TX_I$ is derived from the content of the at least one data packet.

Additionally, prior, during or after the above steps, the actual noise level of the communication unit $I_E$ (in dBm) is determined by the processor of the external device, for example as moving arithmetic mean value. Other methods of determining the mean/average value of the different detected noise level values may be used instead. The noise level $I_E$ may be determined continuously or at pre-defined time intervals for a pre-defined time interval. Based on a known attenuation of the patient's tissue $D_K$, e.g. $D_K = 31.5$ dB, the noise level $I_I$ (in dBm) of the transceiver of the IMD is determined by the processor of the external device using formula II. If, as in the above example, $D_K = 31.5$ dB $I_I$ may be calculated as follows: $I_I = I_E - 12.5$.

Afterwards, $\Delta_E$ and $\Delta_I$ are determined by the processor of the external device using above formulas IIIa and IIIb. In the next step, $C_{Eeff}$ and $C_{Ieff}$ are determined as described in formulas IVa and IVb. Then, the processor determines the highest possible uplink data rate value $d_{UL}$ for data transmission in the uplink direction from table 1 (stored in the processor or a connected data memory) based on $C_{Eeff}$ and a highest possible downlink data rate value $d_{DL}$ for data transmission in the downlink direction from table 1 based on $C_{Ieff}$. The details for above method and the assumptions are explained in detail above.

In one embodiment, the highest possible uplink data rate values for data transmission in the uplink direction and in the downlink direction consider a bit error rate (BER) of $10^{-2}$, $10^{-3}$ or $10^{-5}$ and/or a predetermined type of IMD transceiver module and/or a predetermined frequency band for data transmission. In principle, the method/system applies to every selected bit error rate (BER), with $10^{-3}$ being a useful example in communication systems with normal link. Other useful examples are a BER of $10^{-5}$ in communication systems with a good link and $10^{-2}$ in communication systems with a bad link. Thus, preferably a BER between $10^{-1}$ and $10^{-6}$, and particularly preferably between $10^{-2}$ and $10^{-5}$, is therefore selected.

In one embodiment of the method, the communication unit of the external device transmits the determined highest possible uplink data rate value and the highest possible downlink data rate value to the transceiver module of the IMD and the IMD sets the uplink and downlink data rates subsequently usable by the transceiver module to the data rate values received from the communication unit.

In one embodiment of the method, the external device sets the uplink and downlink data rates subsequently usable by the communication unit to the determined highest possible downlink data rate value and the determined highest possible uplink data rate value as indicated above.

In one embodiment of the method, the processor of the external device determines a signal loss in the data transmission in the uplink and downlink direction as explained above.

The above method is, for example, realized as a computer program (to be executed at the communication device and/or the IMD, in particular at their processors) which is a combination of above and below specified computer instructions and data definitions that enable computer hardware to perform computational or control functions or which is a syntactic unit that conforms to the rules of a particular programming language and that is composed of declarations and statements or instructions needed for an above and below specified function, task, or problem solution.

Furthermore, a computer program product is disclosed comprising instructions which, when executed by a processor, cause the processor to perform the steps of the above defined method. Accordingly, a computer readable data carrier storing such computer program product is described.

The present invention will now be described in further detail with reference to the accompanying schematic drawing, wherein

Fig. 1  shows an embodiment of the communication system comprising an implantable leadless pacemaker (ILP) and a communication unit, wherein the ILP is shown within a cross section of a patient's heart,

Fig. 2  shows the BER (in dB) for typical receiver over energy per bit to noise power spectral density ratio $E_b/N_0$ (in dB), and

Fig. 3  the system of Fig. 1 and an interfering source I.

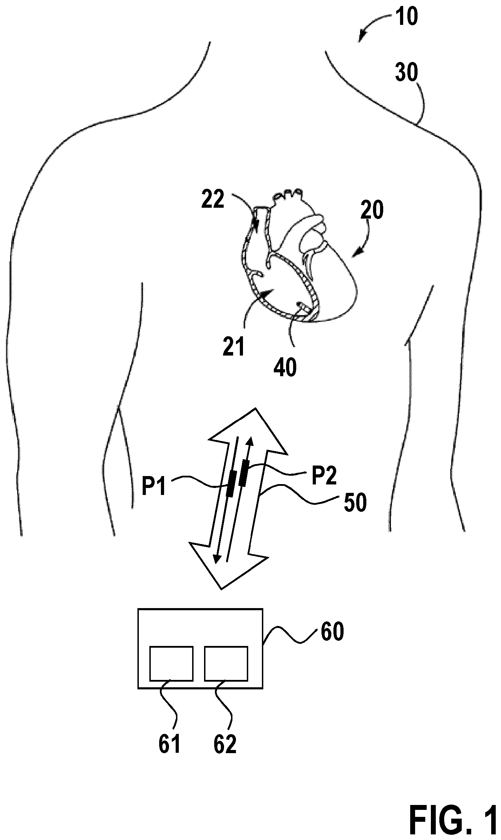

Fig. 1 shows an example communication system 10 and a heart 20 (with right ventricle 21 and right atrium 22) of a patient 30. The system 10 comprises a leadless ventricular pacemaker device 40 (hereinafter "ILP 40") as an example for an IMD and an external device 60. ILP 40 may be configured to be implanted within the right ventricle 21 of the heart (as shown in Fig. 1) and to pace this ventricle, sense intrinsic ventricular depolarizations and impedance, and inhibit ventricular pacing in response to detected ventricular depolarization. The ILP 40 further comprises a transceiver module for sending messages to and receiving signals from a communication unit 62 of the external device 60. The transceiver module comprises, for example, a transceiver chip. The ILP 40 may further comprise an accelerometer sensor in order to measure posture of the patient. The ILP 40 comprises modules such as a processor, a data memory module, a signal generator unit for providing treatment signals (e.g. pacing signals), a measurement unit comprising an ECG measuring unit and a DC impedance sensor. Further, it comprises a power source wherein the modules are electrically connected to each other. The power source may include a battery, e.g., a rechargeable or non-rechargeable battery. The data memory module may include any memory type mentioned above.

The external device 60, such as a smartphone, a computer or programmer is located outside the body and is adapted to communicate bi-directionally with the ILP 40 using its communication unit 62. The external device 60, which can be a portable patient device, comprises a processor 61 and the communication unit 62 containing a transceiver chip for exchange of messages in the form of data packets with the transceiver module of the ILP 40, wherein the processor 61 and the communication unit 62 are electrically connected with each other. The bi-directional exchange of messages (data packets) with the ILP 40 is symbolized by the double arrow 50 in Fig. 1. The wireless communication of the external device 60 with the ILP 40 may be facilitated by electromagnetic waves in the radio-frequency range, for example using an RF band, e.g. MICS band ranging from 402 - 405 MHz. For that, the communication unit 62 and the ILP 40 each comprise a transceiver chip.

The bi-directional communication of the ILP 40 and the external device 60 comprises, for example, far field telemetry communication, wherein only a few data packets containing for example arrhythmia information recorded in the ILP are exchanged between the communication unit 62 of the external device 60 and the transceiver module of the ILP 40.

In order to increase longevity of the ILP 40 it is necessary to communicate with the highest possible data rate in the uplink and downlink direction, wherein the uplink direction is from the transceiver module of the ILP 40 to the communication unit 62 of the external device 60 and the opposite direction is the downlink direction. In the first step one single initial data packet P1 is sent by the transceiver module of the ILP 40 to the communication unit 62 of the external device 60 with a low data rate, for example 4 kb/s, via a pre-defined channel in the MICS band in order to have it decoded by the communication unit 62 in any case. The information of the transmitter power output $TX_I$ is either previously known to the external device (i.e. it is always the same for a known type or class of IMD) or it is provided within the content of the data packet P1, e.g. $TX_I = 0$ dBm.. The communication unit 62 and/or the processor 61 determines the signal strength $C_E$ of this first data packet P1, for example $C_E = -87$ dBm which is a typical value for a transceiver of an IMD operating at a MICS band. Approximately 75% of MICS communication works at a higher signal strength, whereas 25% at a lower signal strength.

Further, it is known that the transmitter power output $TX_E$ of the communication unit 62 is about - 16 dBm so that according to above formula I the following is calculated by the processor 61 with regard to the signal strength of the transceiver module

$$C_I = C_E + TX_E - TX_I = -87 \text{ dBm} - 16 \text{ dBm} - 0 \text{ dBm} = -103 \text{ dBm} .$$

Prior the first step explained above the noise level $I_E$ is determined by the processor 61 of the external device, for example over a time period of 5 s to about $I_E = -109$ dBm. This is a typical value (approximately 10 over N, see table 1) for a home application. For use in clinics a value of approximately 20 dB over N may be measured.

It is further known, that in formula II the value DK (in dB) for this specific constellation is 31.5 so that the noise level

$$I_I = I_E - D_K + 19 = -109 \text{ dBm} - 31.5 \text{ dBm} + 19 = -121.5 \text{ dBm}.$$

In this example the patient's body 30 attenuates the interference so that it becomes negligible compared with the thermal noise N.

In the next step the $\Delta$ values are determined by the processor 61 of the external device 60 based on formulas IIIa and IIIb:

$$\Delta_E = \max (-109 + 125 , 6) = 16 \text{ and}$$

$$\Delta_I = \max (-121.5 + 125 , 6) = 6$$

Accordingly, the processor 61 of the external device 60 further calculates using formulas IVa and IVb:

$$C_{Eeff} = -87 - 16 = -103$$

$$C_{Ieff} = -103 - 6 = -109$$

Using table 1 the highest possible data rates are determined by the processor 61 to $d_{UL} = 200$ kb/s and $d_{DL} = 32$ kb/s. The content of table 1 is saved in the processor 61 or a data memory of the external device 60 connected to the processor 61 as a look-up table. Note that for use in clinics with $I_E$ about 20 dB above thermal noise application of the same formulas would give $C_{Eeff} = -113$ and $C_{Ieff} = -116.5$. In this case using table 1 the highest possible data rates would be determined by the processor 61 to $d_{UL} = 4$ kb/s and $d_{DL} = 4$ kb/s.

The above highest possible data rates for uplink and downlink communication are transmitted from the processor 61 of the external device 60 and sent by the communication unit 62 to the transceiver module of the ILP 40 with a second (response) data packet P2 (see Fig. 1). After sending P2 the communication unit 62 switches to the above determined highest possible data rates $d_{UL}$ and $d_{DL}$ for communication with the transceiver module. After receipt of data packet P2 and revealing of the determined highest possible data rates, the transceiver module is switched to and operates with these new data rates when transmitting the further data packets to the communication unit 62.

**Claims**

1. A communication system (10) for wireless data transmission between an implantable medical device (IMD, 40) and an external device (60) comprising a communication unit (62) and a processor (61), said system comprising said IMD (40) and said external device (60), wherein the IMD (40) is configured to monitor a bodily parameter of a patient (30) and/or is configured to deliver a therapy signal to the patient, wherein the IMD comprises a transceiver module configured to exchange the data with the communication unit of the external device in an uplink direction from the transceiver module to the communication unit and in a downlink direction from the communication unit to the transceiver module, wherein the processor of the external device is configured to determine the signal strength ($C_E$) of at least one data packet (P1) received by the communication unit and sent by the transceiver module of the implanted IMD with a pre-determined initial data rate and to determine the noise level ($I_E$) of the communication unit, wherein the processor is further configured to determine a highest possible uplink data rate value ($d_{UL}$) for data transmission in the uplink direction and a highest possible downlink data rate value ($d_{DL}$) for data transmission in the downlink direction based on the immediately previously determined signal strength of the at least one data packet received by the communication unit from the transceiver module and the current noise level of the communication unit.

2. The communication system of claim 1, wherein the highest possible uplink data rate values for data transmission in the uplink direction and in the downlink direction consider a bit error rate (BER) between $10^{-1}$ and $10^{-6}$, preferably a BER of $10^{-2}$, $10^{-3}$ or $10^{-5}$.

3. The communication system of any of the previous claims, wherein the highest possible uplink data rate values for data transmission in the uplink direction and in the downlink direction consider a predetermined type of IMD transceiver module.

4. The communication system of any of the previous claims, wherein the highest possible uplink data rate values for data transmission in the uplink direction and in the downlink direction consider a predetermined frequency band for data transmission.

5. The communication system of any of the previous claims, wherein the communication unit (62) of the external device (60) is further configured to transmit the determined highest possible uplink data rate value and the highest possible downlink data rate value to the transceiver module of the IMD (40) and the IMD is configured to set the uplink and downlink data rates subsequently usable by the transceiver module to the data rate values received from the communication unit.

6. The communication system of any of the previous claims, wherein the external device (60) is configured to set the uplink and downlink data rates subsequently usable by the communication unit (62) to the determined highest possible downlink data rate value and the determined highest possible uplink data rate value.

7. The communication system of any of the previous claims, wherein the processor (61) of the external device (60) is configured to determine a signal loss (L) in the data transmission in the uplink and downlink direction and/or the processor of the external device is configured to observe the noise level of the communication unit (62) over a pre-determined time period in order to identify subsections of the time period in which regularly the noise level is higher than in other subsections.

8. A communication method (10) for a wireless data transmission between an implantable medical device (IMD, 40) and an external device (60) comprising a communication unit (62) and a processor (61), wherein the IMD (40) is adapted to monitor a bodily parameter of a patient (30) and/or is adapted to deliver a therapy signal to the patient after implantation within the patient's body, wherein the IMD comprises a transceiver module exchanging the data with the communication unit of the external device in an uplink direction from the transceiver module to the communication unit and in a downlink direction from the communication unit to the transceiver module, with the following steps:

   • the processor of the external device determines the signal strength ($C_E$) of at least one data packet (P1) received by the communication unit, which was sent by the transceiver module of the implanted IMD with a pre-determined initial data rate,
   • the processor of the external device determines the noise level ($I_E$) of the communication unit, and
   • the processor determines a highest possible uplink data rate value ($d_{UL}$) for data transmission in the uplink direction and a highest possible downlink data rate value ($d_{DL}$) for data transmission in the downlink direction based on the immediately previously determined signal strength of the at least one data packet received by the communication unit from the transceiver module and the current noise level of the communication unit.

9. The communication method of claim 8, wherein the highest possible uplink data rate values for data transmission in the uplink direction and in the downlink direction consider a bit error rate (BER) between $10^{-1}$ and $10^{-6}$, preferably a BER of $10^{-2}$, $10^{-3}$ or $10^{-5}$ and/or a predetermined type of IMD transceiver module and/or a predetermined frequency band for data transmission.

10. The communication method of any of the claims 8 to 9, wherein the communication unit (62) of the external device (60) transmits the determined highest possible uplink data rate value and the highest possible downlink data rate value to the transceiver module of the IMD (40) and the IMD sets the uplink and downlink data rates subsequently usable by the transceiver module to the transmitted data rate values.

11. The communication method of any of the claims 8 to 10, wherein the external device (60) sets the uplink and downlink data rates subsequently usable by the communication unit (62) to the determined highest possible downlink data rate value and the determined highest possible uplink data rate value.

12. The communication method of any of the claims 8 to 11, wherein the processor (61) of the external device (60) determines a signal loss (L) in the data transmission in the uplink and downlink direction and/or observes the noise level of the communication unit (62) over a pre-determined time period in order to identify subsections of the time period in which regularly the noise level is higher than in other subsections.

13. A computer program product comprising instructions which, when executed by the processor (61) of the external device (60), cause the processor to perform the steps of the method according to any of the claims 8 to 12.

14. Computer readable data carrier storing a computer program product according to claim 13.

**Patentansprüche**

1. Kommunikationssystem (10) zur drahtlosen Datenübertragung zwischen einem implantierbaren medizinischen Gerät (IMD, 40) und einem externen Gerät (60), das eine Kommunikationseinheit (62) und einen Prozessor (61) umfasst, wobei das System das IMD (40) und das externe Gerät (60) umfasst, wobei das IMD (40) so konfiguriert ist, dass es einen Körperparameter eines Patienten (30) überwacht und/oder so konfiguriert ist, dass es ein Therapiesignal an den Patienten liefert, wobei das IMD ein Transceivermodul umfasst, das so konfiguriert ist, dass es die Daten mit der Kommunikationseinheit des externen Geräts in einer Uplink-Richtung von dem Transceivermodul zu der Kommunikationseinheit und in einer Downlink-Richtung von der Kommunikationseinheit zu dem Transceivermodul austauscht, wobei der Prozessor des externen Geräts so konfiguriert ist, dass er die Signalstärke ($C_E$) mindestens eines von der Kommunikationseinheit empfangenen und vom Transceivermodul der implantierten IMD mit einer vorbestimmten Anfangsdatenrate gesendeten Datenpakets (P1) bestimmt und den Rauschpegel ($I_E$) der Kommunikationseinheit bestimmt, wobei der Prozessor ferner konfiguriert ist, um einen höchstmöglichen Uplink-Datenratenwert ($d_{UL}$) für die Datenübertragung in der Uplink-Richtung und einen höchstmöglichen Downlink-Datenratenwert ($d_{DL}$) für die Datenübertragung in der Downlink-Richtung auf der Grundlage der unmittelbar zuvor bestimmten Signalstärke des mindestens einen Datenpakets, das von der Kommunikationseinheit von dem Transceivermodul empfangen wurde, und des aktuellen Rauschpegels der Kommunikationseinheit zu ermitteln.

2. Kommunikationssystem nach Anspruch 1, wobei die höchstmöglichen Uplink-Datenratenwerte für die Datenübertragung in der Uplink-Richtung und in der Downlink-Richtung eine Bitfehlerrate (BER) zwischen $10^{-1}$ und $10^{-6}$, vorzugsweise eine BER von $10^{-2}$, $10^{-3}$ oder $10^{-5}$ berücksichtigen.

3. Kommunikationssystem nach einem der vorhergehenden Ansprüche, wobei die höchstmöglichen Uplink-Datenratenwerte für die Datenübertragung in der Uplink-Richtung und in der Downlink-Richtung einen vorbestimmten Typ von IMD-Transceivermodul berücksichtigen.

4. Das Kommunikationssystem nach einem der vorhergehenden Ansprüche, wobei die höchstmöglichen Uplink-Datenratenwerte für die Datenübertragung in der Uplink-Richtung und in der Downlink-Richtung ein vorbestimmtes Frequenzband für die Datenübertragung berücksichtigen.

5. Kommunikationssystem nach einem der vorhergehenden Ansprüche, wobei die Kommunikationseinheit (62) des externen Geräts (60) ferner dazu ausgebildet ist, den ermittelten höchstmöglichen Uplink-Datenratenwert und den höchstmöglichen Downlink-Datenratenwert an das Transceivermodul des IMD (40) zu übertragen, und das IMD dazu ausgebildet ist, die anschließend vom Transceivermodul nutzbaren Uplink- und Downlink-Datenraten auf die von der Kommunikationseinheit empfangenen Datenratenwerte einzustellen.

6. Das Kommunikationssystem nach einem der vorhergehenden Ansprüche, wobei das externe Gerät (60) so konfiguriert ist, dass es die anschließend von der Kommunikationseinheit (62) nutzbaren Uplink- und Downlink-Datenraten auf den ermittelten höchstmöglichen Downlink-Datenratenwert und den ermittelten höchstmöglichen Uplink-Datenratenwert einstellt.

7. Kommunikationssystem nach einem der vorhergehenden Ansprüche, wobei der Prozessor (61) des externen Geräts (60) so konfiguriert ist, dass er einen Signalverlust (L) bei der Datenübertragung in der Uplink- und Downlink-Richtung bestimmt und/oder der Prozessor des externen Geräts so konfiguriert ist, dass er den Rauschpegel der Kommunikationseinheit (62) über einen vorbestimmten Zeitraum beobachtet, um Teilabschnitte des Zeitraums zu identifizieren, in denen der Rauschpegel regelmäßig höher ist als in anderen Teilabschnitten.

8. Kommunikationsverfahren (10) für eine drahtlose Datenübertragung zwischen einem implantierbaren medizinischen Gerät (IMD, 40) und eines externen Geräts (60), die eine Kommunikationseinheit (62) und einen Prozessor (61) umfasst, wobei die IMD (40) angepasst ist, um einen Körperparameter eines Patienten (30) zu überwachen und/oder angepasst ist, um ein Therapiesignal an den Patienten nach der Implantation im Körper des Patienten zu liefern, wobei das IMD ein Transceivermodul umfasst, das die Daten mit der Kommunikationseinheit des externen Geräts in einer Uplink-Richtung von dem Transceivermodul zu der Kommunikationseinheit und in einer Downlink-Richtung von der Kommunikationseinheit zu dem Transceivermodul austauscht, mit den folgenden Schritten:

   • der Prozessor des externen Geräts die Signalstärke ($C_E$) mindestens eines von der Kommunikationseinheit empfangenen Datenpakets (P1) ermittelt, das vom Transceivermodul der implantierten IMD mit einer vorbestimmten Anfangsdatenrate gesendet wurde
   • der Prozessor des externen Geräts den Rauschpegel ($I_E$) der Kommunikationseinheit bestimmt, und
   • der Prozessor einen höchstmöglichen Uplink-Datenratenwert ($d_{UL}$) für die Datenübertragung in der Uplink-Richtung und einen höchstmöglichen Downlink-Datenratenwert ($d_{DL}$) für die Datenübertragung in der Downlink-Richtung auf der Grundlage der unmittelbar zuvor bestimmten Signalstärke des mindestens einen Datenpakets, das von der Kommunikationseinheit von dem Transceivermodul empfangen wurde, und des aktuellen Rauschpegels der Kommunikationseinheit ermittelt.

9. Kommunikationsverfahren nach Anspruch 8, wobei die höchstmöglichen Uplink-Datenratenwerte für die Datenübertragung in Uplink-Richtung und in Downlink-Richtung eine Bitfehlerrate (BER) zwischen $10^{-1}$ und $10^{-6}$, vorzugsweise eine BER von $10^{-2}$, $10^{-3}$ oder $10^{-5}$ und/oder einen vorbestimmten Typ eines IMD-Transceivermoduls und/oder ein vorbestimmtes Frequenzband für die Datenübertragung berücksichtigen.

10. Kommunikationsverfahren nach einem der Ansprüche 8 bis 9, wobei die Kommunikationseinheit (62) des externen Geräts (60) den ermittelten höchstmöglichen Uplink-Datenratenwert und den höchstmöglichen Downlink-Datenratenwert an das Transceivermodul des IMD (40) überträgt und das IMD die anschließend vom Transceivermodul nutzbaren Uplink- und Downlink-Datenraten auf die übertragenen Datenratenwerte einstellt.

11. Kommunikationsverfahren nach einem der Ansprüche 8 bis 10, wobei das externe Gerät (60) die anschließend von der Kommunikationseinheit (62) nutzbaren Uplink- und Downlink-Datenraten auf den ermittelten höchstmöglichen Downlink-Datenratenwert und den ermittelten höchstmöglichen Uplink-Datenratenwert einstellt.

12. Kommunikationsverfahren nach einem der Ansprüche 8 bis 11, wobei der Prozessor (61) des externen Geräts (60) einen Signalverlust (L) bei der Datenübertragung in der Aufwärts- und Downlink-Richtung ermittelt und/oder den Rauschpegel der Kommunikationseinheit (62) über einen vorbestimmten Zeitraum beobachtet, um Teilabschnitte des Zeitraums zu identifizieren, in denen der Rauschpegel regelmäßig höher ist als in anderen Teilabschnitten.

13. Computerprogrammprodukt mit Befehlen, die, wenn sie vom Prozessor (61) des externen Geräts (60) ausgeführt werden, den Prozessor veranlassen, die Schritte des Verfahrens nach einem der Ansprüche 8 bis 12 durchzuführen.

14. Computerlesbarer Datenträger, der ein Computerprogrammprodukt nach Anspruch 13 speichert.

**Revendications**

1. Système de communication (10) destiné à l'émission de données sans fil entre un dispositif médical implantable (IMD, 40) et un dispositif externe (60) comprenant une unité de communication (62) et un processeur (61), ledit système comprenant ledit IMD (40) et ledit dispositif externe (60), dans lequel l'IMD (40) est configuré pour surveiller un paramètre corporel d'un patient (30) et/ou est configuré pour distribuer un signal thérapeutique au patient, dans lequel l'IMD comprend un module d'émetteur-récepteur configuré pour échanger les données avec l'unité de communication du dispositif externe dans un sens de liaison montante depuis le module d'émetteur-récepteur vers l'unité de communication et dans un sens de liaison descendante depuis l'unité de communication vers le module d'émetteur-récepteur, dans lequel le processeur du dispositif externe est configuré pour déterminer l'intensité du signal ($C_E$) d'au moins un paquet de données (P1) reçu par l'unité de communication et envoyé par le module d'émetteur-récepteur de l'IMD implanté avec un débit de données initial prédéterminé et pour déterminer le niveau de bruit ($I_E$) de l'unité de communication, dans lequel le processeur est en outre configuré pour déterminer une valeur de débit de données de liaison montante la plus élevée possible ($d_{UL}$) pour l'émission de données dans le sens de la liaison montante et une valeur de débit de données de liaison descendante la plus élevée possible ($d_{DL}$) pour l'émission de données dans le sens de la liaison descendante en se basant sur l'intensité de signal déterminée immédiatement précédemment de l'au moins un paquet de données reçu par l'unité de communication depuis le module d'émetteur-récepteur et le niveau de bruit actuel de l'unité de communication.

2. Système de communication selon la revendication 1, dans lequel les valeurs de débit de données de liaison montante les plus élevées possibles pour l'émission de données dans le sens de la liaison montante et dans le sens de la liaison descendante prennent en compte un taux d'erreur de bits (BER) compris entre $10^{-1}$ et $10^{-6}$, de préférence un BER de $10^{-2}$, $10^{-3}$ ou $10^{-5}$.

3. Système de communication selon l'une quelconque des revendications précédentes, dans lequel les valeurs de débit de données de liaison montante les plus élevés possibles pour l'émission de données dans le sens de la liaison montante et dans le sens de la liaison descendante prennent en compte un type prédéterminé de module d'émetteur-récepteur d'IMD.

4. Système de communication selon l'une quelconque des revendications précédentes, dans lequel les valeurs de débit de données de liaison montante les plus élevés possibles pour l'émission de données dans le sens de la liaison montante et dans le sens de la liaison descendante prennent en compte une bande de fréquences prédéterminée pour l'émission de données.

5. Système de communication selon l'une quelconque des revendications précédentes, dans lequel l'unité de communication (62) du dispositif externe (60) est en outre configurée pour transmettre la valeur de débit de données de liaison montante la plus élevée possible déterminée et la valeur de débit de données de liaison descendante la plus élevée possible au module d'émetteur-récepteur de l'IMD (40) et l'IMD est configuré pour définir les débits de données de liaison montante et de liaison descendante conséquemment utilisables par le module d'émetteur-récepteur aux valeurs de débit de données reçues depuis l'unité de communication.

6. Système de communication selon l'une quelconque des revendications précédentes, dans lequel le dispositif externe (60) est configuré pour définir les débits de données de liaison montante et de liaison descendante conséquemment utilisables par l'unité de communication (62) à la valeur de débit de données de liaison descendante la plus élevée possible déterminée et la valeur de débit de données de liaison montante la plus élevée possible déterminée.

7. Système de communication selon l'une quelconque des revendications précédentes, dans lequel le processeur (61) du dispositif externe (60) est configuré pour déterminer une perte de signal (L) dans l'émission de données dans le sens de la liaison montante et de la liaison descendante et/ou le processeur du dispositif externe est configuré pour observer le niveau de bruit de l'unité de communication (62) sur une période prédéterminée afin d'identifier les sous-sections de la période dans laquelle le niveau de bruit est régulièrement supérieur à celui des autres sous-sections.

8. Procédé de communication (10) pour une émission de données sans fil entre un dispositif médical implantable (IMD, 40) et un dispositif externe (60) comprenant une unité de communication (62) et un processeur (61), dans lequel l'IMD (40) est adapté pour surveiller un paramètre corporel d'un patient (30) et/ou est adapté pour distribuer un signal thérapeutique au patient après implantation au sein du corps du patient, dans lequel l'IMD comprend un module d'émetteur-récepteur échangeant les données avec l'unité de communication du dispositif externe dans un sens de liaison montante depuis le module d'émetteur-récepteur vers l'unité de communication et dans un sens de liaison descendante depuis l'unité de communication vers le module d'émetteur-récepteur, avec les étapes suivantes :

• le processeur du dispositif externe détermine la force du signal ($C_E$) d'au moins un paquet de données (P1) reçu par l'unité de communication, qui a été envoyé par le module d'émetteur-récepteur de l'IMD implanté avec un débit de données initial prédéterminé,
• le processeur du dispositif externe détermine le niveau de bruit ($I_E$) de l'unité de communication, et
• le processeur détermine une valeur de débit de données de liaison montante la plus élevée possible ($d_{UL}$) pour l'émission de données dans le sens de la liaison montante et une valeur de débit de données de liaison descendante la plus élevée possible ($d_{DL}$) pour l'émission de données dans le sens de la liaison descendante en se basant sur l'intensité du signal déterminée immédiatement précédemment de l'au moins un paquet de données reçu par l'unité de communication depuis le module d'émetteur-récepteur et le niveau de bruit actuel de l'unité de communication.

9.  Procédé de communication selon la revendication 8, dans lequel les valeurs de débit de données de liaison montante les plus élevées possibles pour l'émission de données dans le sens de la liaison montante et dans le sens de la liaison descendante prennent en compte un taux d'erreur de bits (BER) compris entre $10^{-1}$ et $10^{-6}$, de préférence un BER de $10^{-2}$, $10^{-3}$ ou $10^{-5}$ et/ou un type prédéterminé de module d'émetteur-récepteur d'IMD et/ou une bande de fréquences prédéterminée pour l'émission de données.

10. Procédé de communication selon l'une quelconque des revendications 8 à 9, dans lequel l'unité de communication (62) du dispositif externe (60) transmet la valeur de débit de données de liaison montante la plus élevée possible déterminée et la valeur de débit de données de liaison descendante la plus élevée possible au module d'émetteur-récepteur de l'IMD (40) et l'IMD définit les débits de données de liaison montante et de liaison descendante conséquemment utilisables par le module d'émetteur-récepteur aux valeurs de débit de données émises.

11. Procédé de communication selon l'une quelconque des revendications 8 à 10, dans lequel le dispositif externe (60) définit les débits de données de liaison montante et de liaison descendante conséquemment utilisables par l'unité de communication (62) à la valeur de débit de données de liaison descendante la plus élevée possible déterminée et la valeur de débit de données de liaison montante la plus élevée possible déterminée.

12. Procédé de communication selon l'une quelconque des revendications 8 à 11, dans lequel le processeur (61) du dispositif externe (60) détermine une perte de signal (L) dans l'émission de données dans le sens de la liaison montante et de la liaison descendante et/ou observe le niveau de bruit de l'unité de communication (62) sur une période prédéterminée afin d'identifier les sous-sections de la période dans laquelle régulièrement le niveau de bruit est supérieur à celui des autres sous-sections.

13. Produit de programme informatique comprenant des instructions qui, lorsqu'elles sont exécutées par le processeur (61) du dispositif externe (60), amènent le processeur à réaliser les étapes du procédé selon l'une quelconque des revendications 8 à 12.

14. Support de données lisible par ordinateur stockant un produit de programme informatique selon la revendication 13.

FIG. 1

FIG. 2

FIG. 3

**EP 4 115 946 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030009204 A1
- US 20180152972 A1